(19) Europäisches Patentamt / European Patent Office / Office européen des brevets

(11) **EP 2 427 227 B1**

(12) **EUROPEAN PATENT SPECIFICATION**

(45) Date of publication and mention
of the grant of the patent:
**10.08.2016 Bulletin 2016/32**

(51) Int Cl.:
*A61L 29/14* *(2006.01)*          *A61L 29/16* *(2006.01)*
*A61L 29/08* *(2006.01)*

(21) Application number: **10719554.7**

(86) International application number:
**PCT/US2010/032900**

(22) Date of filing: **29.04.2010**

(87) International publication number:
**WO 2010/129381 (11.11.2010 Gazette 2010/45)**

(54) **BALLOON COATING WITH DRUG TRANSFER CONTROL VIA COATING THICKNESS**

BALLONBESCHICHTUNG MIT WIRKSTOFFÜBERTRAGUNGSREGELUNG ÜBER DIE BESCHICHTUNGSDICKE

REVÊTEMENT POUR BALLON PERMETTANT DE RÉGULER LE TRANSFERT D'UN MÉDICAMENT GRÂCE À L'ÉPAISSEUR DU REVÊTEMENT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.05.2009 US 437401**

(43) Date of publication of application:
**14.03.2012 Bulletin 2012/11**

(73) Proprietor: **Abbott Cardiovascular Systems Inc.**
**Abbott Park, IL 60064-6008 (US)**

(72) Inventors:
• **STANKUS, John, J.**
**Campbell**
**CA 95008 (US)**
• **PACETTI, Stephen, D.**
**San Jose**
**CA 95130 (US)**

(74) Representative: **Jackson, Martin Peter**
**J A Kemp**
**14 South Square**
**Gray's Inn**
**London WC1R 5JJ (GB)**

(56) References cited:
**WO-A1-2007/065722     WO-A1-2009/051614**

• **BURKE S E ET AL: "Zotarolimus (ABT-578) eluting stents" ADVANCED DRUG DELIVERY REVIEWS, ELSEVIER BV, AMSTERDAM, NL, vol. 58, no. 3, 3 June 2006 (2006-06-03), pages 437-446, XP024892174 ISSN: 0169-409X DOI: DOI:10.1016/J.ADDR.2006.01.021 [retrieved on 2006-06-03]**
• **B. SCHELLER ET AL.: "Treatment of Coronary In-Stent Restenosis with a Paclitaxel-Coated Balloon Catheter" THE NEW ENGLAND JOURNAL OF MEDICINE, vol. 355, no. 20, 16 November 2008 (2008-11-16), pages 2113-2124, XP002614505**
• **MAIER L S ET AL: "Hotline update of clinical trials and registries presented at the German Cardiac Society meeting 2008; (PEPCAD, LokalTax, INH, German ablation registry, German device registry, DES.DE registry, DHR, Reality, SWEETHEART registry, ADMA, GERSHWIN)" CLINICAL RESEARCH IN CARDIOLOGY, STEINKOPFF-VERLAG, DA, vol. 97, no. 6, 25 April 2008 (2008-04-25), pages 356-363, XP019628416 ISSN: 1861-0692**

**Description**

**CROSS-REFERENCE TO RELATED APPLICATIONS**

This present application claims the benefit of U.S. Non-Provisional Application No. 12/437,401, filed May 7, 2009.

**FIELD OF THE INVENTION**

The present invention is related to the delivery of drugs from an insertable medical device. More particularly, the present invention relates to a coated balloon having a coating thickness exhibiting improved coating transfer efficiency and/or uptake of therapeutic agent to a blood vessel wall.

**BACKGROUND OF THE INVENTION**

Atherosclerosis is a syndrome affecting arterial blood vessels. It is a chronic inflammatory response in the walls of arteries, which is in large part due to the accumulation of lipid, macrophages, foam cells and the formation of plaque in the arterial wall. Atherosclerosis is commonly referred to as hardening of the arteries although the pathophysiology of the disease manifests itself with several different types of lesions ranging from fibrotic to lipid laden to calcific. Angioplasty is a vascular interventional technique involving mechanically widening an obstructed blood vessel, typically caused by atherosclerosis.

During angioplasty, a catheter having a tightly folded balloon is inserted into the vasculature of the patient and is passed to the narrowed location of the blood vessel at which point the balloon is inflated to a fixed size using fluid pressures. Percutaneous coronary intervention (PCI), commonly known as coronary angioplasty, is a therapeutic procedure to treat the stenotic coronary arteries of the heart, often found in coronary heart disease. In contrast, peripheral angioplasty, commonly known as percutaneous transluminal angioplasty (PTA), refers to the use of mechanical widening of blood vessels other than the coronary arteries. PTA is most commonly used to treat narrowing of the leg arteries, especially, the iliac, external iliac, superficial femoral and popliteal arteries. PTA can also treat narrowing of veins, and other blood vessels.

Although the blood vessel is often successfully widened by angioplasty, sometimes the treated wall of the blood vessel undergoes vasospasm, or abrupt closure after balloon inflation or dilatation, causing the blood vessel to collapse after the balloon is deflated or shortly thereafter. One solution to such collapse is stenting the blood vessel to prevent collapse. A stent is a device, typically a metal tube or scaffold, that is inserted into the blood vessel after, or concurrently with angioplasty, to hold the blood vessel open.

While the advent of stents eliminated many of the complications of abrupt vessel closure after angioplasty procedures, within about six months of stenting a re-narrowing of the blood vessel often formed, a condition known as restenosis. Restenosis was discovered to be a response to the injury of the angioplasty procedure and is characterized by a growth of smooth muscle cells-analogous to a scar forming over an injury. It was thought that drug eluting stents were the answer to the reoccurrence of the narrowing of blood vessels after stent implantation. A drug eluting stent is a metal stent that has been coated with a drug that is known to interfere with the process of re-narrowing of the blood vessel (restenosis). It was then discovered that a drawback of drug eluting stents was a condition known as late stent thrombosis, which is an event in which blood clots inside the stent. Stent thrombosis, whether acute or late, can be fatal in over one-third of cases.

Drug eluting balloons are believed to be a viable alternative to drug eluting stents in the treatment of atherosclerosis. In a study which evaluated restenosis, and the rate of major adverse cardiac events such as heart attack, bypass, repeat stenosis, or death in patients treated with drug eluting balloons and drug eluting stents, the patients treated with drug eluting balloons experienced only 3.7 percent restenosis and 4.8% MACE (material adverse coronary events) as compared to patients treated with drug eluting stents, in which restenosis was 20.8 percent and 22.0 percent MACE rate. (See, PEPCAD II study, Rotenburg, Germany).

Although drug eluting balloons are a viable alternative, and in some cases appear to have greater efficacy than drug eluting stents as suggested by the PEPCAD II study, drug eluting balloons present unique challenges. In particular, the drug needs to be released from the balloon surface or the coating needs to be transferred to the blood vessel wall when the balloon is expanded inside the blood vessel. For coronary procedures, the balloon is typically inflated for less than one minute, typically about thirty seconds. The balloon may be able to be expanded for a longer period of time for peripheral procedure, however typically even for peripheral procedures the balloon is expanded for less than 5 minutes. Due to the very short duration of contact of the drug coated balloon surface with the blood vessel wall, the balloon coating must exhibit optimal therapeutic agent transfer efficiency and/or efficient drug release during inflation which is within minutes. Thus, there are challenges specific to drug delivery via a drug coated (or drug eluting) balloon because of the necessity of a short inflation time, and therefore time for drug or coating transfer--a challenge not presented by a drug eluting stent, which remains in the patient's vasculature once implanted.

WO2009/051614 discloses drug delivery balloons having a coating of 2 $\mu$m.

## SUMMARY OF INVENTION

The present invention includes a drug delivery balloon which exhibits improved coating transfer efficiency to the wall of a blood vessel and/or increased uptake of therapeutic agent into a blood vessel wall. The balloon of the invention has a coating applied to at least a portion of the balloon surface. The coating has a thickness of about 1.5 to about 10 $\mu$m. Preferably, the coating has a thickness is of about 2 to about 6 $\mu$m. It has surprisingly been found that a drug delivery balloon having such a coating thicknesses exhibits greater coating transfer efficiency and therapeutic uptake.

The coating includes a therapeutic agent and has a thickness between about 1.5 and 10 $\mu$m, preferably between about 2 and 6 $\mu$m. Surprisingly, less than 30% of the coating remains on the balloon post delivery, inflation and deflation, or post removal from a lumen of a subject. Preferably, less than 20% and more preferably less than 10% of the coating remains on the balloon post delivery, inflation and deflation, or post removal from a lumen of a subject.

The therapeutic agent is zotarolimus. In one embodiment, the dosage of therapeutic agent is about 15 ug/cm$^2$ to about 600 ug/cm$^2$.

The coating includes an excipient. The excipient is preferably less than 75% or less than 50% by weight of the coating. The excipient can have hydrophilic properties and binder properties. The excipient includes polyvinyl pyrrolidone (PVP).

Preferably, the PVP is not substantially cross-linked, and is not a hydrogel. In one embodiment, the PVP has a molecular weight of less than 60 kilodaltons. In yet another embodiment, the PVP has a molecular weight of less than 30 kilodaltons. In accordance with one embodiment, polyethylene glycol has a molecular weight less than 1000 daltons.

The coating further includes glycerol The coating includes zotarolimus, PVP, and glycerol. In one embodiment, the weight ratio of the zotarolimus:PVP:glycerol is about 20:1 to 1:2 for zotarolimus:PVP, preferably, is about 1:1 to 1:0.1 for PVP:glycerol and more preferably is about 2:1:0.4 for zotarolimus:PVP:glycerol. In another embodiment (not part of the invention) the coating includes zotarolimus and a non-ionic contrast agent, such as but not limited to an iopromide. In one embodiment, the iopromide is Ultravist. The weight ratio of the zotarolimus:non-ionic contrast agent is about 10:1 to 1:10 and more preferably about 2:1, such as 1.95:1.

Preferably, the coated balloon is disposed on a catheter body for insertion of the drug delivery balloon to the vasculature of a patient. The catheter can include an elongate tubular member having a proximal end, a distal end and a lumen there between. In one embodiment, the catheter has an over-the-wire configuration. In another embodiment, catheter has a rapid exchange configuration.

In accordance with another aspect of the invention, a coated medical device is provided, such as a balloon including a stent. The medical device includes an expandable member having a surface and a coating applied to at least a portion of the surface of the expandable member. The coating comprises a therapeutic agent and an excipient and has a thickness of about 1.5 to 10 $\mu$m.

In yet another aspect of the invention, a method of manufacturing a drug delivery balloon is provided. In this regard, the method includes applying a coating to at least a portion of an expandable member to define a thickness of about 1.5 to 10 um, and preferably from 2 to 6 um, and disposing the expandable member on a catheter. The method can further include the step of preparing a pre-coating mixture for example by mixing a therapeutic agent and an excipient according to claim 8 and conditioning the pre-coating to form a porous coating by a phase inversion technique. Additionally, or alternatively, the method can include the step of creating a coating to which a porogen is added to define a porous coating for application to the medical device.

In one embodiment, the porous coating is created by phase inversion techniques. In another embodiment, the porous coating is created by introduction of a porogen to a mixture including a therapeutic agent to be applied to the delivery device. In one embodiment, the porogen is removed from the coating prior to application of the coating to the delivery device.

It is to be understood that both the foregoing description is exemplary and is intended to provide further explanation of the invention claimed to a person of ordinary skill in the art. The accompanying drawings are included to illustrate various embodiments of the invention to provide a further understanding of the invention. The exemplified embodiments of the invention are not intended to limit the scope of the claims.

## BRIEF DESCRIPTION OF DRAWINGS

Figure 1 depicts one embodiment of a medical device of the invention;

Figure 2 is a graph illustrating the results from a comparative study of drug delivery balloons and coating transfer efficiency in a porcine coronary and mammary pharmacokinetic model;

Figure 3 is a graph illustrating percent drug remaining on post delivery balloons as a function of theoretical coating thicknesses of drug delivery balloons having varied formulations;

Figure 4 is a graph illustrating therapeutic agent and percent initial balloon dose remaining in tissue after delivery in a porcine coronary and mammary pharmacokinetic model using an embodiment of the present invention;

Figure 5 depicts one embodiment of a medical device of the invention.

## DETAILED DESCRIPTION

Reference will now be made in detail to the present embodiments of the invention, an example of which is illustrated in the accompanying figures. The invention will be described in conjunction with the detailed description of the device. However, no intent to limit the scope of the invention to the specific embodiments described exists.

The device and method of the invention may be used for treating the lumen of a patient. In particular, the invention is particularly suited for treatment of the cardiovascular system of a patient, such as performance of angioplasty and/or delivery of a coated expandable medical balloon in the coronary or peripheral blood vessels.

In accordance with one aspect of the invention, a balloon for delivering a therapeutic agent according to claim 1 is provided.

In one embodiment, less than 10% of the coating remains on the balloon post delivery into a lumen of a subject. That is, at least 90% of the coating is delivered from the balloon or medical device. In another embodiment, less than 30% of the coating remains on the balloon after inflation and deflation in the lumen of a subject. In yet another embodiment, less than 30% of the coating remains on the balloon post removal of the balloon or medical device from the lumen of the subject. Preferably, less than 20% of the coating remains on the balloon post delivery, inflation and deflation, and/or removal from a lumen of a subject. More preferably, less than 10% of the coating remains on the balloon post delivery, inflation and deflation, and/or removal from a lumen of a subject.

The therapeutic agent is zotarolimus.

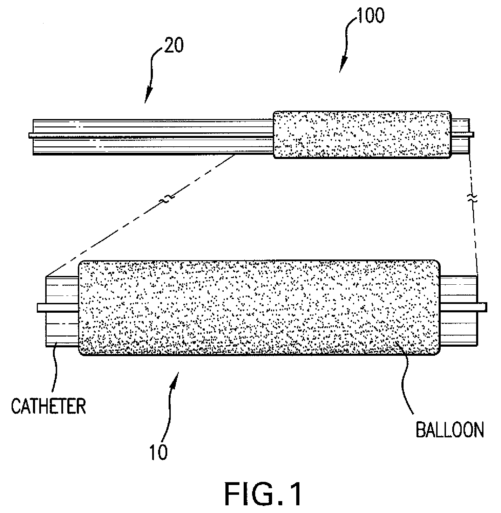

Referring to Figure 1, a device 100 is provided drug delivery balloon 10 that exhibits improved coating transfer from the balloon and/or therapeutic agent uptake to a blood vessel wall is provided. In one embodiment, the balloon 10 is disposed on a catheter 20, as shown in Figure 1. In this regard, it has been surprisingly discovered that a balloon having a coating thickness of about 1.5 to 10 $\mu$m and preferably 2 to 6 $\mu$m exhibits improved coating transfer efficiency. In one embodiment, less than 30% of the initial coating remains on the balloon post delivery to a lumen in a subject. In another embodiment, less than 30% of the coating remains on the balloon or at least a portion of the balloon post inflation and deflation in a lumen of a subject. In yet another embodiment, less than 30% of the coating remains on the balloon post removal from a subject. Accordingly, more than 70% of the coating transfers from the balloon to the subject. Preferably, less than 20 % of the coating remains on the balloon, and more preferably less than 10% of the coating remains on the balloon.

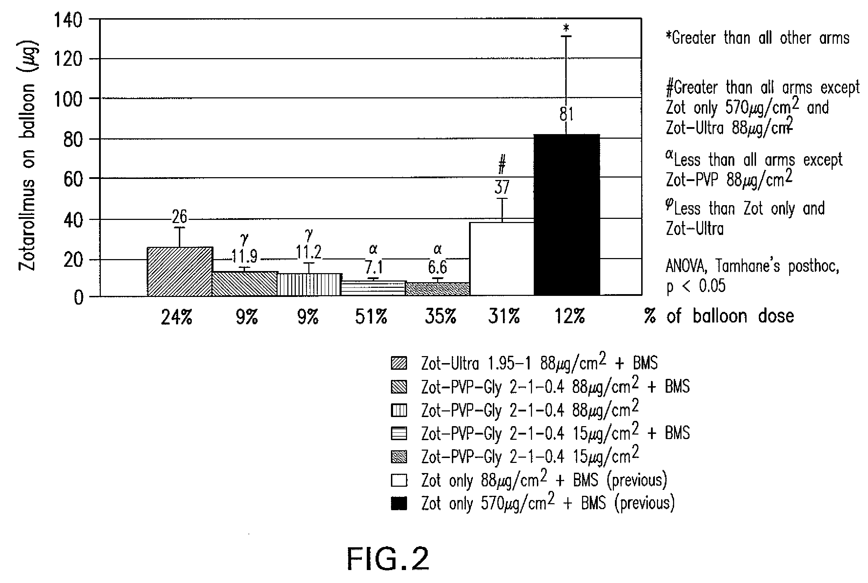

Figure 2 shows the results from a comparative study in which seven different coated balloons were delivered to healthy porcine coronary or mammary arteries in pharmacokinetic models. The coating formulations are tabulated in Table 1 Balloon 1, 4-7 are not part of the invention.

| TABLE 1 | | |
|---|---|---|
| **Balloon** | **Formulation** | **Dosage of Therapeutic Agent** |
| 1 | Zotarolimus: Ultravist (1.95:1 weight ratio) | 88 $\mu$g/cm$^2$ |
| 2 | Zotarolimus: PVP: Glycerol (2:1:0.4 weight ratio) | 88 $\mu$g/cm$^2$ |
| 3 | Zotarolimus: PVP: Glycerol (2:1:0.4 weight ratio) | 8 $\mu$g/cm$^2$ (no stent) |
| 4 | Zotarolimus: PVP: Glycerol (2:1:0.4 weight ratio) | 15 $\mu$g/cm$^2$ |
| 5 | Zotarolimus: PVP: Glycerol (2:1:0.4 weight ratio) | 15 $\mu$g/cm$^2$ (no stent) |
| 6 | Zotarolimus | 88 $\mu$g/cm$^2$ |
| 7 | Zotarolimus | 570 $\mu$g/cm$^2$ |

All of the coatings include a therapeutic agent. Most of the coating formulations include excipients or different doses of therapeutic agent to achieve varied coating thickness on the balloons. The drug delivery balloons were inserted and inflated for 30 seconds in the animal model. Thereafter, the drug delivery balloons were withdrawn and then the percentage of the initial drug dosage remaining on the balloon surface was calculated. The remaining drug on each of the balloons was assayed by extraction of the balloons in an organic solvent mixture followed by analysis using high pressure liquid chromatography (HPLC).

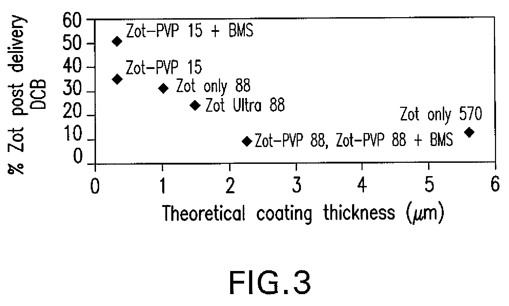

It was surprisingly found that the balloons having thicker coatings exhibited greater coating transfer efficiency from the balloon to the blood vessel wall. In particular, as depicted in Figure 2, Balloons 6 and 7 (counting from left to right) are each coated with pure zotarolimus. Balloons 6 and 7 are not part of the invention. The zotarolimus coating applied to Balloon 7 has a dose density of 570 $\mu$g/cm$^2$ of zotarolimus, and the coating applied to Balloon 6 has 88 $\mu$g/cm$^2$ of zotarolimus. As shown in Figure 3, the theoretical thicknesses for the coatings was calculated to be about 1 $\mu$m for Balloon 6 and about 6 $\mu$m for Balloon 7. This theoretical thickness was calculated based on the mass and density of the coating and balloon surface area via the formula:

$$T = \frac{W}{A\rho} = \frac{V_{coating}}{A}$$

where T = average coating thickness
W = coating mass
A = coated balloon area
$\rho$ = coating density (assumed 1.1 gm/cm$^3$)
$V_{coating}$ = coating volume

As shown, Balloon 7 has a greater coating transfer efficiency than does Balloon 6. In particular, the percentage of coating transfer for Balloon 6 is 69%, whereas the balloon coating transfer for Balloon 7 is 88%.

Likewise, Balloons 2 and 3 each have coating formulations comprising zotarolimus, PVP, and glycerol. The dosage of zotarolimus is 88 $\mu$g/cm$^2$ and the drug:PVP:Glycerol is in a ratio of 2:1:0.4. In contrast, Balloons 4 and 5 (which are not part of the invention) also have a coating of zotarolimus, PVP, and glycerol in a 2:1:0.4 ratio. However, the dosage of zotarolimus in Balloons 4 and 5 is 15 $\mu$g/cm$^2$. As shown in Figure 3, the theoretical coating thicknesses of Balloons 2 and 3 are 2.25 $\mu$m, whereas the theoretical coating thicknesses for Balloons 4 and 5 are 0.5 $\mu$m. Balloons 2 and 3 both exhibit over 90% coating transfer efficiency, while Balloons 4 and 5 exhibit less than 65% coating transfer, as shown in Figure 2. Thus, the balloons having thicker coatings resulted in improved coating transfer efficiency.

Referring to Figure 2, Balloon 1 has a coating formulation of zotarolimus and Ultravist in a ratio of 1.95:1 (w/w). The theoretical coating thickness of Balloon 1 is about 1.5 $\mu$m, as shown in Figure 3. Balloon 1 exhibited a coating transfer of about 76%, which is a greater coating transfer efficiency than the Balloons 4 and 5 having a coating thickness of about 0.5 $\mu$m, but a lesser coating transfer efficiency than Balloons 2 and 3 which exhibited 90% coating transfer efficiency.

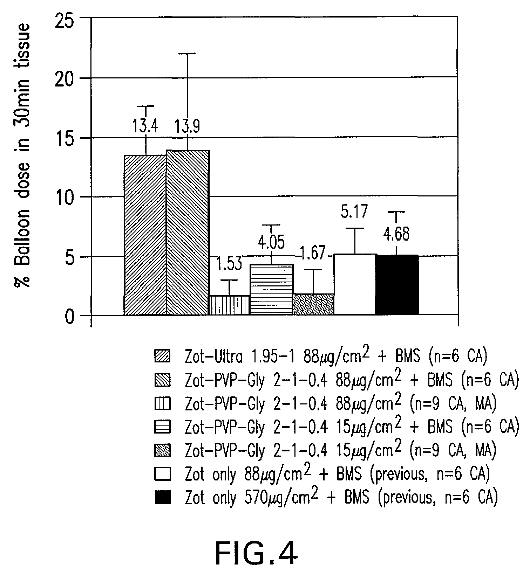

In another aspect of the invention, a drug delivery balloon is provided which exhibits improved tissue uptake of therapeutic agent. Figure 4 shows the results from a comparative study in which various drug delivery balloons having the formulations of Table 1 were inserted and inflated in porcine coronary and mammary artery pharmacokinetic models. The drug delivery balloons were inserted via femoral access and delivered to either the LCX, LAD, RCA, LIMA or RIMA arteries for a thirty second inflation. After deflation of the balloon and removal, the balloons were clipped and frozen until HPLC analysis. The percent of zotarolimus dose per the original balloon dose transferred to the tissue 30 minutes after balloon inflation is depicted in the graph of Figure 4.

As shown in Figure 4, Balloon 2 and Balloon 4 (not part of the invention) both have formulations of zotarolimus: PVP: glycerol. The coatings differ in that Balloon 4 has zotarolimus in an amount of 15 $\mu$g/cm$^2$ and Balloon 2 has zotarolimus in an amount of 88 $\mu$g/cm$^2$. Consequently, the coating of Balloon 2 is thicker than the coating of Balloon 4. As shown in Figure 4, Balloon 2 exhibits greater tissue uptake of zotarolimus than does Balloon 4. Thus, it appears drug delivery balloons having a thicker coating improves drug uptake into the tissue of the vessel wall.

Further, it was surprisingly found that the tissue uptake has greater improvements when the drug delivery balloon includes a stent crimped on the balloon. In this regard, comparison of Balloon 2 and Balloon 3, each of which have identical coating formulations, exhibited different drug uptake into the tissues of the vessel walls. In particular, Balloon 2 which includes a bare metal stent crimped on the balloon during delivery exhibited greater than six-fold increase in zotarolimus tissue uptake than did Balloon 3, which has no stent disposed on the drug delivery balloon.

Likewise, Balloon 4 and Balloon 5 (which are not part of the invention) each include identical coating formulations, except that Balloon 4 further includes a bare metal stent disposed on the balloon and Balloon 5 has no stent. As shown in Figure 4, the inclusion of a stent crimped on the Balloon 4 resulted in a greater than two-fold increase in zotarolimus uptake by the tissue as compared to Balloon 5. Thus, in addition to coating thicknesses, the inclusion of a bare metal stent disposed on the drug delivery balloon improves tissue uptake of therapeutic agent. Thus, in another aspect of the invention, a drug delivery balloon is provided which exhibits improved tissue uptake of therapeutic agent in one aspect of the invention. The drug delivery balloon comprises a coating applied to at least a portion of the balloon surface and a stent disposed on balloon. In this regard, the stent can be a bare metal stent, a coated stent or a drug eluting stent.

In accordance with the invention, the coating can be applied to a medical device by processes such as dip-coating, pipette coating, syringe coating, air assisted spraying, electrostatic spraying, piezoelectric spraying, electrospinning, direct fluid application, or other means as known to those skilled in the art. The coating may contain the drug homogeneously dissolved or encapsulated in particles. The coating can be applied over at least a portion or the entirety of the balloon or medical device. By way of example, and not limitation, certain coating processes that may be used with the instant invention are described in U.S. Patent No. 6,669,980 to Hansen; U.S. Patent No. 7,241,344 to Worsham; and U.S. Publication No. 2004/0234748 to Stenzel. The medical device of the invention is a balloon and the coating can be applied to either a folded or inflated balloon. Coating characteristics are affected by process variables. For example, for a dip-coating process, coating quality and thickness can vary as an effect of variables such as number of dips, rate of withdrawal, and depth of dips along with drying time and temperature.

In accordance with another aspect of the invention, a method of manufacturing a drug delivery device is provided. The drug delivery device is a balloon. The method includes applying a coating including an effective amount of the therapeutic agent to an expandable member to define a coating thickness of about 1.5 to about 10 $\mu$m, and disposing the expandable member on a catheter. In an alternative embodiment, the method includes providing a catheter including an expandable member; and applying a coating including an effective amount of the therapeutic agent to the expandable member to define a coating thickness of about 1.5 to about 10 $\mu$m. The catheter includes an elongate shaft having a proximal end, a distal end and at least one lumen therebetween. Preferably, the catheter includes a multilumen shaft such as an inflation lumen and a guidewire lumen. In this regard, multilumen can be arranged in a coaxial or side-by-side configuration. Further, the catheter can be configured as a rapid exchange catheter or an over-the-wire catheter.

The method can further include the step of preparing the coating, during which the preparation step includes mixing the therapeutic agent, such as an effective amount therapeutic agent, and the excipient to form a precoating, and conditioning the precoating by a phase inversion technique to define a porous coating for application to the expandable member. Alternatively, or additionally, the method can include defining a porous coating by adding a porogen to the coating or preparing the coating by inclusion of a porogen, as described below.

In accordance with the invention, the coating thickness applied to a medical device or a balloon is controlled. Various techniques are available to control the coating thickness for a drug delivery balloon. For the purpose of illustration but not limitation, the coating thickness can be controlled by changing: (1) drug dose per unit of balloon surface area, (2) percent solids of drugs and excipients in the coating solution, (3) ratio of therapeutic agent to excipients in the drug formulation, (4) changing the surface area of coating per a certain dose and formulation, (5) adding porosity or void volume of the coating, or (6) particulars of the coating process such as coating method, drying rate and solvent used.

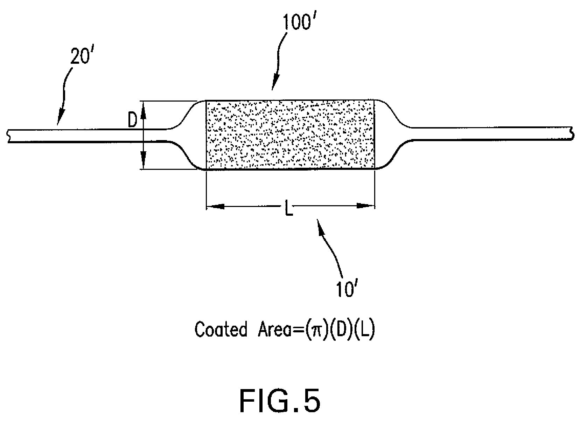

In one embodiment, the coating thickness is controlled for a given therapeutic agent dose and formulation. For example and for the purpose of illustration but not limitation, Figure 5 shows that the coated area for drug delivery balloon 10 can be calculated by the following equation: Coated Area = $(\pi)(D)(L)$; where D is the diameter of the balloon and L is the working length or coated length of the balloon.

For example, the surface area may be reduced by decreasing length (L) of the balloon for a particular therapeutic agent dose and formulation. Rather than decreasing the working area of the balloon that is coated, the balloon may be coated by a series of bands wrapping around the balloon, or stripes running along the length of the balloon. Many other patterns are possible such as checkerboard or a plurality of dots. In all of these cases, the amount of drug dissolution from the balloon, rate of drug dissolution, or coating transfer to the vessel wall will be increased via an increase in coating thickness.

Other means to increase the coating thickness include: (1) increasing the therapeutic agent dose, and (2) increasing the amount of excipient for a given drug dose. While increasing the dose will render the coating more prone to fracture, during inflation there is an upper limit on the amount of therapeutic agent that can be used so as not to exceed the no observable adverse effect level ("NOAEL"), which is based on systemic drug exposure and available toxicological data for the drug.

In addition, a porous coating of the same dose would have a larger coating thickness. There are many methods to create a porous, open celled coating such as (1) incorporation of a porogen into the coating, which is subsequently leached out after the coating process (e.g., salt leaching) and (2) use of a coating which undergoes phase inversion (e.g., thermal induced phase separation). Phase inversion is a process that creates porous structures. Phase inversion either starts with a homogenous single phase solution (Sol 1) which at some point before gelation undergoes a transition into a heterogeneous solution of molecular aggregates consisting of two interdispersed liquid phases (Sol 2), or it starts with a heterogeneous solution of molecular aggregates consisting of two interdispersed liquid phases (Sol 2).

Phase inversion can be accomplished by use of a solvent and excipient blends in a drying process, a thermal process where the polymer is only soluble at an elevated temperature in the solvent, or a wet process where a dense coating is subsequently exposed to additional solvent processing. The drying process is most applicable to coatings containing a drug. A simple concept is to dissolve the drug and excipients in a solvent blend where the faster evaporating solvent is compatible solvents for the polymer/drug. Other examples of phase inversion techniques to produce porous surfaces include lyophilization, high pressure gas foaming, solid freeform fabrication, fiber bonding of extruded microfibers and fiber based electrospinning of micro- or nano-fibers.

In accordance with the invention, the balloon is a polymeric expandable balloon. Various polymers may be selected for the formation of the balloon, as would be known in the art. For example, the polymeric material may be may be a compliant, non-compliant or semi-compliant polymeric material or polymeric blend.

In one embodiment, the polymeric material is compliant such as but not limited to a polyamide/polyether block copolymer (commonly referred to as PEBA or polyether-block-amide). Preferably, the polyamide and polyether segments of the block copolymers may be linked through amide or ester linkages. The polyamide block may be selected from various aliphatic or aromatic polyamides known in the art. Preferably, the polyamide is aliphatic. Some non-limiting examples include nylon 12, nylon 11, nylon 9, nylon 6, nylon 6/12, nylon 6/11, nylon 6/9, and nylon 6/6. Preferably, the polyamide is nylon 12. The polyether block may be selected from various polyethers known in the art. Some non-limiting examples of polyether segments include poly(tetramethylene glycol), tetramethylene ether, polyethylene glycol, polypropylene glycol, poly(pentamethylene ether) and poly(hexamethylene ether). Commercially available PEBA material may also be utilized such as for example, PEBAX® materials supplied by Arkema (France). Various techniques for forming a balloon from polyamide/polyether block copolymer are known in the art. One such example is disclosed in US 6,406,457 to Wang.

In another embodiment, the balloon material is formed from polyamides. Preferably, the polyamide has substantial tensile strength, be resistant to pin-holing even after folding and unfolding, and be generally scratch resistant, such as those disclosed in US 6,500,148 to Pinchuk. Some non-limiting examples of polyamide materials suitable for the balloon include nylon 12, nylon 11, nylon 9, nylon 69 and nylon 66. Preferably, the polyamide is nylon 12. In yet another embodiment, the balloon is composed of several different layers, each a one a different polyamide or polyamide/polyether block copolymer.

In another embodiment, the balloon may be formed a polyurethane material, such as TECOTHANE® (Thermedics). TECOTHANE® is a thermoplastic, aromatic, polyether polyurethane synthesized from methylene disocyanate (MDI), polytetramethylene ether glycol (PTMEG) and 1,4 butanediol chain extender. TECOTHANE® grade 1065D is presently preferred, and has a Shore durometer of 65D, an elongation at break of about 300%, and a high tensile strength at yield of about 10,000 psi. However, other suitable grades may be used, including TECOTHANE® 1075D, having a Shore D of 75. Other suitable compliant polymeric materials include ENGAGE® (DuPont Dow Elastomers (an ethylene alpha-olefin polymer) and EXACT® (Exxon Chemical), both of which are thermoplastic polymers. Other suitable compliant materials include, but are not limited to, elastomeric silicones, latexes, and urethanes. The compliant material may be cross linked or uncrosslinked, depending upon the balloon material and characteristics required for a particular application. The presently preferred polyurethane balloon materials are not crosslinked. However, other suitable materials, such as the polyolefinic polymers ENGAGE® and EXACT®, are preferably crosslinked. By crosslinking the balloon compliant material, the final inflated balloon size can be controlled. Conventional crosslinking techniques can be used including thermal treatment and E-beam exposure. After crosslinking, initial pressurization, expansion, and preshrinking, the balloon will thereafter expand in a controlled manner to a reproducible diameter in response to a given inflation pressure, and thereby avoid overexpanding the stent (when used in a stent delivery system) to an undesirably large diameter. In one embodiment, the balloon is formed from a low tensile set polymer such as a silicone-polyurethane copolymer. Preferably, the silicone-polyurethane is an ether urethane and more specifically an aliphatic ether urethane such as PURSIL AL 575A and PURSIL AL10, (Polymer Technology Group), and ELAST-EON 3-70A, (Elastomedics), which are silicone polyether urethane copolymers, and more specifically, aliphatic ether urethane cosiloxanes. In an alternative embodiment, the low tensile set polymer is a diene polymer. A variety of suitable diene polymers can be used such as but not limited to an isoprene such as an AB and ABA poly(styrene-block-isoprene), a neoprene, an AB and ABA poly(styrene-block-butadiene) such as styrene butadiene styrene (SBS) and styrene butadiene rubber (SBR), and 1,4-polybutadiene. Preferably, the diene polymer is an isoprene including isoprene copolymers and isoprene block copolymers such as poly(styrene-block-isoprene). A presently preferred isoprene is a styrene-isoprene-styrene block copolymer, such as Kraton 1161K available from Kraton, Inc. However, a variety of suitable isoprenes can be used including HT 200 available from Apex Medical, Kraton R 310 available from Kraton, and isoprene (i.e., 2-methyl-1,3-butadiene) available from Dupont Elastomers. Neoprene grades useful in the invention include HT 501 available from Apex Medical, and neoprene (i.e., polychloroprene) available from Dupont Elastomers, including Neoprene G, W, T and A types available from Dupont Elastomers.

In accordance with the invention, the balloon can be composed of a single polymeric layer, or alternatively, can be a multilayered balloon, such as those described in U.S. Patent No. 5,478,320 to Ishida, U.S. Patent No. 5,879,369 to Trotta, or U.S. Patent No. 6,620,127 to Lee.

In one embodiment, the outer surface of the balloon is textured. In this regard, the balloon surface may include a roughened surface, voids, spines, or microcapsules or a combination thereof, as will be described below.

In one embodiment of the invention, the balloon is formed of a porous elastomeric material having at least one void formed in the wall of the balloon surface. The entire cross section of the balloon may contain a plurality of voids. Alternatively, the plurality of void may be distributed along select portions of the balloon outer surface. For example and not limitation, the plurality of voids can be distributed only along only the working section of the balloon. The voids define an open space within the outer surface of the balloon. Preferably, the therapeutic agent is dispersed within the space defined by the plurality of voids across the cross section of the balloon outer surface.

In operation, the therapeutic agent is released or is expelled from the pores upon inflation of the balloon. In this regard, the durometer of the polymeric material of the balloon surface and in particular the depression of the void is sufficiently flexible to allow for expulsion of the therapeutic agent and/or coating contained within the plurality of voids upon inflation of the balloon. The expelled coating with therapeutic agent is released into the vessel lumen or into the tissue surrounding and contacting the inflated balloon.

In another embodiment, as embodied herein, the balloon includes protrusions configured to contact or penetrate the arterial wall of a vessel upon inflation of the balloon. A coating containing therapeutic agent is disposed on the protrusions and when inflated the coating and/or therapeutic agent coats the tissue of the arterial wall. Alternatively, the balloon may include two concentric balloons in a nesting configuration. The coating with therapeutic agent is disposed between the two concentric balloons. Thus, the space between the two concentric balloons; one being an interior balloon and the other being an exterior balloon, acts as a reservoir. In this regard, the protrusions may include apertures for expulsion of the coating and/or therapeutic agent upon inflation of the interior and exterior concentric balloons. For example, as described in US 6,991,617 to Hektner. In another embodiment, the balloon may include longitudinal protrusions configured to form ridges on the balloon surface. As described in US 7,273,417 to Wang, the ridges can be formed of filaments spaced equidistantly apart around the circumference of the balloon. However, a larger or smaller number of ridges can alternatively be used. The longitudinal ridges can be fully or partially enveloped by the polymeric material of the balloon.

In yet another embodiment of the invention, the balloon may include microcapsules on its outer surface. In this regard, the microcapsules are configured to encompass the coating and/or therapeutic agent. Upon inflation of the balloon the microcapsules located on the surface of the balloon contact the tissue of the arterial wall. Alternatively, the microcapsules may be formed in the wall of the balloon surface. The coating and/or therapeutic agent may be released from the microcapsules by fracturing of the microcapsules and/or diffusion from the microcapsule into the arterial wall. The microcapsules may be fabricated in accordance with the methods disclosed in US 5,1023,402 to Dror or US 6,129,705 to Grantz and the patents referenced therein.

In accordance with another aspect of the invention, if desired, a protective sheath may be utilized to protect the coating from being rubbed off of the balloon during the movement of the coated balloon through the body lumen. The sheath is preferably made from an elastic and resilient material which conforms to the shape of the balloon and in particular is capable of expanding upon inflation of the balloon. The sheath preferably includes apertures along a portion thereof. In operation, the inflation of the balloon causes the apertures of the sheath to widen for release of the coating and/or therapeutic agent to the tissue of the arterial wall. Preferably, the sheath has a thickness less than 10 mils. However, other thicknesses are possible.

In another embodiment, the sheath has at least one longitudinal line of weakness allowing the sheath to rupture upon inflation of the balloon and the release of the coating and/or therapeutic agent onto the tissue of the arterial wall of the vessel. Preferably, the sheath is formed from polymeric material known to be suitable for use in balloon catheters. Preferably, the sheath material is an elastomeric material which will also spring back when it splits to expose more of the body lumen to the coating. The line of weakness could be provided by various techniques known in the art. However, one non-limiting examples include perforating the sheath material. In operation, the sheath is placed over the coated balloon while in the deflated state. When the coated balloon inflated, the sheath is expanded to the extent that it exceeds its elastic limit at the line of weakness and bursts to expose and therefore release the coating and/or therapeutic agent to the tissue of the arterial wall or vessel lumen. For example, see US 5,370,614 to Amundson.

In accordance with another aspect of the invention, a coated balloon is provided. The medical device comprises an expandable member having a surface and a coating having a thickness of about 2 to about 6 um is applied to the surface of the expandable member. The coating has a thickness of about 2 to about 6 um.

## Claims

1.  A balloon for delivering a therapeutic agent to a vessel wall of a subject, the balloon comprising:

    a body having a working portion disposed between distal and proximal ends thereof; and
    a coating applied to at least a portion of the balloon, wherein the coating comprises zotarolimus, polyvinyl pyrrolidone, and glycerol, the coating having a coating thickness of between 1.5 $\mu$m to 10 $\mu$m.

**2.** The balloon of claim 1, wherein the weight ratio of zotarolimus:polyvinyl pyrrolidone is from 20:1 to 1:2.

**3.** The balloon of claim 2, wherein the weight ratio of polyvinyl pyrrolidone:glycerol is from 1:1 to 1:0.1.

**4.** The balloon of claim 1, wherein the weight ratio of zotarolimus: polyvinyl pyrrolidone: glycerol is 2:1:0.4.

**5.** The balloon of claim 1, wherein a stent is disposed on the balloon.

**6.** The balloon of claim 1, wherein zotarolimus has a dose density on the balloon of between 15 $\mu$g/cm$^2$ to 600 $\mu$g/cm$^2$.

**7.** The balloon of claim 1, wherein the coating thickness is defined by controlling dose density of the therapeutic agent on the balloon, controlling the ratio of the therapeutic agent and the excipient in the coating, or forming the coating in a pattern on the balloon body.

**8.** A method of manufacturing a drug delivery device for delivering a therapeutic agent to a vessel wall of a subject, comprising:

providing a catheter comprising a balloon having a balloon body, the balloon body including a working portion disposed between distal and proximal ends thereof; and
applying a coating to the balloon body defining a coating thickness of between 1.5 $\mu$m to 10 $\mu$m, the coating comprising an effective amount of a therapeutic agent which is zotarolimus, polyvinyl pyrrolidone, and glycerol.

**9.** The method of claim 8, wherein the coating further comprises a porogen, and the method optionally further comprises preparing the coating by mixing a therapeutic agent and an excipient to form a precoating and conditioning the precoating to form a porous coating by a phase inversion technique.

**10.** The method of claim 8, further comprising disposing a stent on the balloon.

**Patentansprüche**

**1.** Ballon zur Abgabe eines therapeutischen Mittels an eine Gefäßwand eines Subjektes, wobei der Ballon umfasst:

einen Körper mit einem Arbeitsteil, angeordnet zwischen den distalen und proximalen Enden davon, und
eine Beschichtung, die auf mindestens einem Teil des Ballons aufgebracht ist, wobei die Beschichtung Zotarolimus, Polyvinyl-pyrrolidon und Glycerol umfasst und die Beschichtung eine Beschichtungsdicke von zwischen 1,5 $\mu$m bis 10 $\mu$m hat.

**2.** Der Ballon nach Anspruch 1, worin das Gewichtsverhältnis Zotarolimus:Polyvinyl-pyrrolidon von 20:1 bis 1:2 ist.

**3.** Der Ballon nach Anspruch 2, worin das Gewichtsverhältnis Polyvinyl-pyrrolidon:Glycerol von 1:1 bis 1:0,1 ist.

**4.** Der Ballon nach Anspruch 1, worin das Gewichtsverhältnis Zotarolimus: Polyvinyl-pyrrolidon:Glycerol 2:1:0,4 ist.

**5.** Der Ballon nach Anspruch 1, worin ein Stent auf dem Ballon angeordnet ist.

**6.** Der Ballon nach Anspruch 1, worin Zotarolimus eine Dosierungsdichte auf dem Ballon von zwischen 15 $\mu$g/cm$^2$ bis 600 $\mu$g/cm$^2$ hat.

**7.** Der Ballon nach Anspruch 1, worin die Beschichtungsdichte definiert wird durch die Steuerung der Dosierungsdichte des therapeutischen Mittels auf dem Ballon, Steuerung des Verhältnisses des therapeutischen Mittels und des Hilfsstoffs in der Beschichtung, oder Bildung der Beschichtung in einem Muster auf dem Ballonkörper.

**8.** Verfahren zur Herstellung einer Arzneimittelabgabevorrichtung zur Abgabe eines therapeutischen Mittels an eine Gefäßwand eines Subjektes umfassend:

Bereitstellen eines Katheters umfassend einen Ballon mit einem Ballonkörper, wobei der Ballonkörper einen Arbeitsbereich einschließt, der zwischen den distalen und proximalen Enden davon angeordnet ist; und

Aufbringen einer Beschichtung auf den Ballonkörper, definierend eine Beschichtungsdicke von zwischen 1,5 $\mu$m bis 10 $\mu$m, wobei die Beschichtung, die Zotarolimus, Polyvinyl-pyrrolidon und Glycerol ist, eine wirksame Menge eines therapeutischen Mittels umfasst.

**9.** Das Verfahren nach Anspruch 8, wobei die Beschichtung weiterhin ein Porogen aufweist und das Verfahren gegebenenfalls weiterhin umfasst die Herstellung der Beschichtung durch Vermischen eines therapeutischen Mittels und eines Hilfsstoffs, so dass eine Vorbeschichtung gebildet wird und die Vorbeschichtung konditioniert wird, so dass eine poröse Beschichtung mittels einer Phaseninversionstechnik gebildet wird.

**10.** Das Verfahren nach Anspruch 8, weiterhin umfassend das Anordnen eines Stents auf dem Ballon.

**Revendications**

**1.** Ballonnet servant à apporter un agent thérapeutique à une paroi de vaisseau chez un sujet, lequel ballonnet comprend :

   - un corps comportant une partie opérante située entre ses extrémités proximale et distale,
   - et un revêtement appliqué sur au moins une partie du ballonnet, lequel revêtement contient du zotarolimus, de la poly(vinyl-pyrroli-done) et du glycérol, et présente une épaisseur de 1,5 à 10 $\mu$m.

**2.** Ballonnet conforme à la revendication 1, dans lequel le rapport pondéral zotarolimus/poly(vinyl-pyrrolidone) vaut de 20/1 à 1/2.

**3.** Ballonnet conforme à la revendication 2, dans lequel le rapport pondéral poly(vinyl-pyrrolidone)/glycérol vaut de 1/1 à 1/0,1.

**4.** Ballonnet conforme à la revendication 1, dans lequel les rapports pondéraux zotarolimus/poly(vinyl-pyrrolidone)/glycérol valent 2/1/0,4.

**5.** Ballonnet conforme à la revendication 1, sur lequel ballonnet est disposé un stent.

**6.** Ballonnet conforme à la revendication 1, dans lequel la densité de la dose de zotarolimus sur le ballonnet vaut de 15 à 600 $\mu$g/cm$^2$.

**7.** Ballonnet conforme à la revendication 1, pour lequel on définit l'épaisseur du revêtement en ajustant la densité de la dose de l'agent thérapeutique sur le ballonnet, en ajustant le rapport de l'agent thérapeutique et de l'excipient dans le revêtement, ou en donnant au revêtement, sur le corps du ballonnet, la forme d'un motif.

**8.** Procédé de fabrication d'un dispositif d'apport de médicament servant à apporter un agent thérapeutique à une paroi de vaisseau chez un sujet, lequel procédé comporte les étapes suivantes :

   - prendre un cathéter comportant un ballonnet comprenant un corps de ballonnet, lequel corps de ballonnet comporte une partie opérante située entre ses extrémités proximale et distale,
   - et appliquer un revêtement sur le corps de ballonnet, en donnant au revêtement une épaisseur de 1,5 $\mu$m à 10 $\mu$m, lequel revêtement comprend, en une quantité efficace, un agent thérapeutique qui est du zotarolimus, ainsi que de la poly(vinyl-pyrrolidone) et du glycérol.

**9.** Procédé conforme à la revendication 8, dans lequel le revêtement comprend en outre un agent porogène, et lequel procédé comporte en outre le fait de préparer le revêtement en mélangeant un agent thérapeutique et un excipient pour en faire un pré-revêtement, et le fait de conditionner ce pré-revêtement pour en faire un revêtement poreux par une technique d'inversion de phases.

**10.** Procédé conforme à la revendication 8, qui comporte en outre le fait de disposer un stent sur le ballonnet.

FIG.1

FIG.2

FIG.3

FIG.4

Coated Area=$(\pi)(D)(L)$

FIG.5

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 43740109 A
- WO 2009051614 A
- US 6669980 B
- US 7241344 B, Worsham
- US 20040234748 A, Stenzel
- US 6406457 B, Wang
- US 6500148 B, Pinchuk
- US 5478320 A, Ishida
- US 5879369 A, Trotta
- US 6620127 B, Lee
- US 6991617 B, Hektner
- US 7273417 B, Wang
- US 51023402 B
- US 6129705 A, Grantz
- US 5370614 A, Amundson